# EUROPEAN PATENT APPLICATION

(11) **EP 1 690 555 A2**
(43) Date of publication of application: **16.08.2006**
(21) Application number: 06250512.8
(22) Date of filing: 31.01.2006
(51) Int. Cl.: A61L 15/60

(54) **Absorbent articles comprising polyamine-coated superabsorbent polymers**

(30) Priority: 01.02.2005 US 49050
(71) Applicant: Kimberly-Clark Worldwide, Inc., Neenah, WI 54956 (US)
(72) Inventor: Laumer, Jason Matthew, Appleton Wisconsin 45915 (US); Miatudila, Ma-Ikay Kikama, Monroe North Carolina 28110 (US); Herfert, Norbert, Charlotte North Carolina 28266 (US); Mitchell, Michael A., Waxhaw North Carolina 28173 (US)
(74) Representative: Beacham, Annabel Rose

(57) **Abstract**

An absorbent article comprises a fluid pervious topsheet, a fluid impervious backsheet which may be joined to the topsheet, and an absorbent core positioned between the topsheet and the backsheet. Additionally, the absorbent core comprises at least a superabsorbent material comprising a base polymer having a surface coating comprising a polyammonium carbonate. The result is an absorbent article which exhibits improved performance as well as greater comfort and confidence among the user.

## Description

### BACKGROUND

Absorbent articles are useful for absorbing many types of fluids, including fluids secreted or eliminated by the human body. Superabsorbent materials are frequently used in absorbent articles to help improve the absorbent properties of such articles. Superabsorbent materials are generally polymer based and are available in many forms, such as powders, granules, microparticles, films and fibers, for example. Upon contact with fluids, such superabsorbent materials swell by absorbing the fluids into their structures. In general, superabsorbent materials can quickly absorb fluids insulted into such articles, and can retain such fluids to prevent leakage and help provide a dry feel even after fluid insult.

There is continuing effort to improve the performance of such absorbent articles, especially at high levels of fluid saturation, to thereby reduce the occurrence of leakage. This is particularly significant when such articles are subjected to repeated fluid insults during use. This has become an increasing challenge as recent efforts in absorbent article design have generally focused on using higher concentrations of superabsorbent material and less fluff fibers to make the absorbent structures thinner and denser. However, notwithstanding the increase in total absorbent capacity obtained by increasing the concentration of superabsorbent material, such absorbent articles may still nevertheless leak during use. Such leakage may in part be the result of the absorbent core having an insufficient intake rate (i.e., the rate at which a fluid insult can be taken into and entrained within the absorbent core for subsequent absorption by the superabsorbent material) due to lack of available void volume. Therefore, there is a desire for an absorbent article which contains high levels of superabsorbent materials and which maintains a sufficient intake rate.

Additionally, increasing the performance of one absorbent property of an absorbent article can often result in an adverse effect on other absorbent properties. For example, as alluded to above, an increase in fluid intake rate can often result in a decrease in capacity. While an increase in fluid intake rate is generally desirable, a corresponding decrease in capacity is generally undesirable. Therefore, there is an additional desire for an absorbent article which exhibits an improved fluid intake rate without adversely affecting other absorbent properties such as capacity. Furthermore, there is a desire to accomplish such an improvement without resorting to complex, capital-intensive absorbent fabrication processes or additional non-absorbent binder fiber components. Such means should be compatible with conventional, low cost, efficient air-forming equipment that is widely used in the industry and integrated in the absorbent article manufacturing process.

### SUMMARY

The present invention concerns an absorbent article, suitably a disposable absorbent article, such as a training pant. More particularly, the absorbent article comprises a topsheet, a backsheet and an absorbent core positioned between the topsheet and the backsheet. Additionally, the absorbent core comprises at least a superabsorbent material which comprising a base polymer having a surface coating comprising a polyammonium carbonate. The result is an absorbent article which exhibits improved performance as well as greater comfort and confidence among the user.

Numerous other features and advantages of the present invention will appear from the following description. In the description, reference is made to exemplary embodiments of the invention. Such embodiments do not represent the full scope of the invention. Reference should therefore be made to the claims herein for interpreting the full scope of the invention.

### FIGURES

The foregoing and other features, aspects and advantages of the present invention will become better understood with regard to the following description, appended claims and accompanying drawings where:
FIG 1 is a perspective view of one embodiment of an absorbent article that may be made in accordance with the present invention;
FIG 2 is a plan view of the absorbent article shown in FIG 1 with the article in an unfastened, unfolded and laid flat condition showing the surface of the article that faces the wearer when worn and with portions cut away to show underlying features;
FIG 3 is a schematic diagram of one version of a method and apparatus for producing an absorbent core; and
FIG 4 is a cross-sectional side view of a layered absorbent core according to the present invention.

Repeated use of reference characters in the present specification and drawings is intended to represent the same or analogous features or elements of the present invention.

### DEFINITIONS

It should be noted that, when employed in the present disclosure, the terms "comprises," "comprising" and other derivatives from the root term "comprise" are intended to be open-ended terms that specify the presence of any stated features, elements, integers, steps, or components, and are not intended to preclude the presence or addition of one or more other features, elements, integers, steps, components, or groups thereof.

The term "absorbent article" generally refers to devices which can absorb and contain fluids. For example, personal care absorbent articles refer to devices which are placed against or near the skin to absorb and contain the various fluids discharged from the body. The term "disposable" is used herein to describe absorbent articles that are not intended to be laundered or otherwise restored or reused as an absorbent article after a single use. Examples of such disposable absorbent articles include, but are not limited to, personal care absorbent articles, health/medical absorbent articles, and household/industrial absorbent articles.

The term "coform" is intended to describe a blend of meltblown fibers and cellulose (eg wood) fibers that is formed by air forming a meltblown polymer material while simultaneously blowing air-suspended cellulose fibers into the stream of meltblown fibers. The coform material may also include other materials, such as superabsorbent materials. The meltblown fibers containing cellulose fibers are collected on a forming surface, such as provided by a foraminous belt. The forming surface may include a gas-pervious material, such as spunbonded fabric material, that has been placed onto the forming surface.

The terms "elastic," "elastomeric" and "elastically extensible" are used interchangeably to refer to a material or composite that generally exhibits properties which approximate the properties of natural rubber. The elastomeric material is generally capable of being extended or otherwise deformed, and then recovering a significant portion of its shape after the extension or deforming force is removed.

The term "extensible" refers to a material that is generally capable of being extended or otherwise deformed, but which does not recover a significant portion of its shape after the extension or deforming force is removed.

The term "fluid impermeable," when used to describe a layer or laminate, means that fluid such as water or bodily fluids will not pass substantially through the layer or laminate under ordinary use conditions in a direction generally perpendicular to the plane of the layer or laminate at the point of fluid contact.

The term "health/medical absorbent article" includes a variety of professional and consumer health-care products including, but not limited to, products for applying hot or cold therapy, medical gowns (i.e., protective and/or surgical gowns), surgical drapes, caps, gloves, face masks, bandages, wound dressings, wipes, covers, containers, filters, disposable garments and bed pads, medical absorbent garments, underpads, and the like.

The term "household/industrial absorbent articles" include construction and packaging supplies, products for cleaning and disinfecting, wipes, covers, filters, towels, disposable cutting sheets, bath tissue, facial tissue, nonwoven roll goods, home-comfort products including pillows, pads, mats, cushions, masks and body care products such as products used to cleanse or treat the skin, laboratory coats, cover-alls, trash bags, stain removers, topical compositions, pet care absorbent liners, laundry soil/ink absorbers, detergent agglomerators, lipophilic fluid separators, and the like.

The terms "hydrophilic" and "wettable" are used interchangeably to refer to a material having a contact angle of water in air of less than 90 degrees. The term "hydrophobic" refers to a material having a contact angle of water in air of at least 90 degrees. For the purposes of this application, contact angle measurements are determined as set forth in Robert J. Good and Robert J. Stromberg, Ed., in "Surface and Colloid Science - Experimental Methods," Vol. II, (Plenum Press, 1979).

The term "insult target zone" refers to an area of an absorbent core where it is particularly desirable for the majority of a fluid insult, such as urine, menses, or bowel movement, to initially contact. In particular, for an absorbent core with one or more fluid insult points in use, the insult target zone refers to the area of the absorbent core extending a distance equal to 15% of the total length of the core from each insult point in both directions.

The term "materials" when used in the phrase "superabsorbent materials" refers generally to discrete units. The units can comprise particles, granules, fibers, flakes, agglomerates, rods, spheres, needles, particles coated with fibers or other additives, pulverized materials, powders, films, and the like, as well as combinations thereof. The materials can have any desired shape such as, for example, cubic, rod-like, polyhedral, spherical or semi-spherical, rounded or semi-rounded, angular, irregular, etc. Additionally, superabsorbent materials may be composed of more than one type of material.

The term "meltblown fibers" refers to fibers formed by extruding a molten thermoplastic material through a plurality of fine, usually circular, die capillaries as molten threads or filaments into a high velocity, usually heated, gas (e.g., air) stream which attenuates the filaments of molten thermoplastic material to reduce their diameter. Thereafter, the meltblown fibers are carried by the high velocity gas stream and are deposited on a collecting surface to form a web of randomly disbursed meltblown fibers.

The terms "nonwoven" and "nonwoven web" refer to materials and webs of material having a structure of individual fibers or filaments which are interlaid, but not in an identifiable manner as in a knitted fabric. The terms "fiber" and "filament" are used herein interchangeably. Nonwoven fabrics or webs have been formed from many processes such as, for example, meltblowing processes, spunbonding processes, air laying processes, and bonded-carded-web processes. The basis weight of nonwoven fabrics is usually expressed in ounces of material per square yard (osy) or grams per square meter (gsm) and the fiber diameters are usually expressed in microns. (Note that to convert from osy to gsm, multiply osy by 33.91.)

The term "personal care absorbent article" includes, but is not limited to, absorbent articles such as diapers, diaper pants, baby wipes, training pants, absorbent underpants, child care pants, swimwear, and other disposable garments; feminine care products including sanitary napkins, wipes, menstrual pads, menstrual pants, panty liners, panty shields, interlabials, tampons, and tampon applicators; adult-care products including wipes, pads such as breast pads, containers, incontinence products, and urinary shields; clothing components; bibs; athletic and recreation products; and the like.

The term "polymers" includes, but is not limited to, homopolymers, copolymers, such as for example, block, graft, random and alternating copolymers, terpolymers, etc. and blends and modifications thereof. Furthermore, unless otherwise specifically limited, the term "polymer" shall include all possible configurational isomers of the material. These configurations include, but are not limited to isotactic, syndiotactic and atactic symmetries.

The terms "spunbond" and "spunbonded fiber" refer to fibers which are formed by extruding filaments of molten thermoplastic material from a plurality of fine, usually circular, capillaries of a spinneret, and then rapidly reducing the diameter of the extruded filaments.

The term "stretchable" refers to materials which may be extensible or which may be elastically extensible.

The terms "superabsorbent" and "superabsorbent materials" refer to water-swellable, water-insoluble organic or inorganic materials capable, under the most favourable conditions, of absorbing at least about 10 times their weight, or at least about 15 times their weight, or at least about 25 times their weight in an aqueous solution containing 0.9 weight percent sodium chloride. In contrast, "absorbent materials" are capable, under the most favourable conditions, of absorbing at least 5 times their weight of an aqueous solution containing 0.9 weight percent sodium chloride.

These terms may be defined with additional language in the remaining portions of the specification.

### DETAILED DESCRIPTION

The present invention concerns an absorbent article, suitably a disposable absorbent article, such as a training pant. More particularly, the absorbent article comprises a topsheet which may be fluid pervious, a backsheet which may be fluid impervious and that may be joined to the topsheet, and an absorbent core positioned between the topsheet and the backsheet. Additionally, the absorbent core comprises at least a superabsorbent material comprising a base polymer having a surface coating comprising a polyammonium carbonate. The result is an absorbent article which exhibits improved performance as well as greater comfort and confidence among the user.

In some embodiments, at least the topsheet can be stretchable, while in other embodiments, at least the backsheet can be stretchable. In still other embodiments, the absorbent core can be stretchable. The absorbent article may also include other components, such as fluid wicking layers, intake layers, surge layers, distribution layers, transfer layers, barrier layers, wrapping layers and the like, as well as combinations thereof.

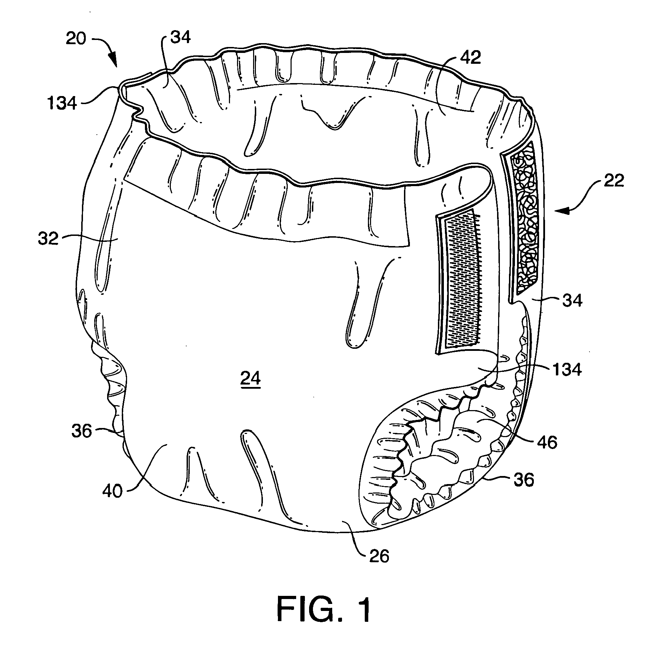

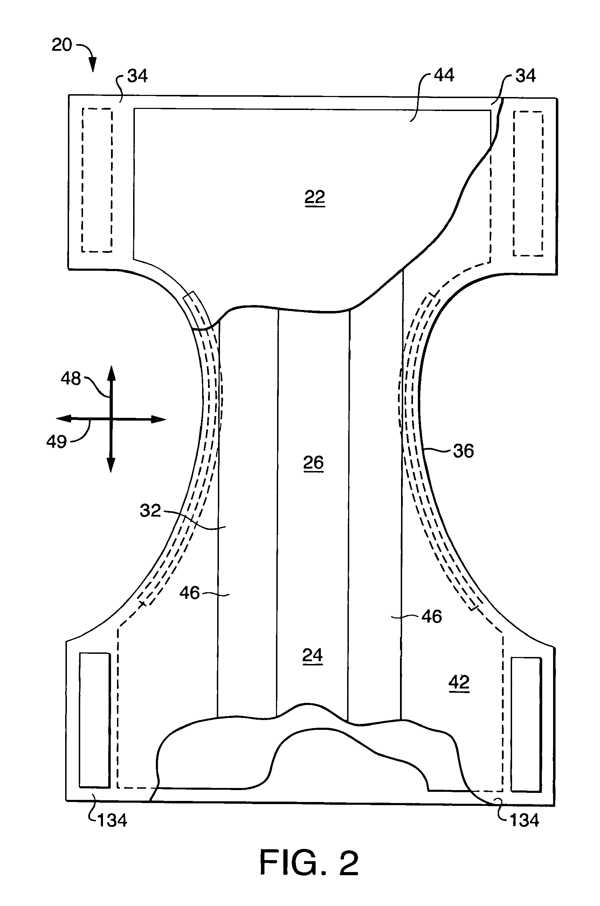

Referring to FIGs 1 and 2 for exemplary purposes, a training pant which may incorporate the present invention is shown. It is understood that the present invention is suitable for use with various other absorbent articles, including but not limited to other personal care absorbent articles, health/medical absorbent articles, household/industrial absorbent articles, and the like without departing from the scope of the present invention.

Various materials and methods for constructing training pants are disclosed in PCT Patent Application WO 00/37009 published June 29, 2000 by A. Fletcher et al.; U.S. Patents 4,940,464 to Van Gompel et al.; 5,766,389 to Brandon et al., and 6,645,190 to Olson et al.

FIG 1 illustrates a training pant in a partially fastened condition, and FIG 2 illustrates a training pant in an opened and unfolded state. The training pant defines a longitudinal direction 48 that extends from the front of the training pant when worn to the back of the training pant. Perpendicular to the longitudinal direction 48 is a lateral direction 49.

The pair of training pants defines a front region 22, a back region 24 and a crotch region 26 extending longitudinally between and interconnecting the front and back regions. The pant also defines an inner surface adapted in use (e.g., positioned relative to the other components of the pant) to be disposed toward the wearer, and an outer surface opposite the inner surface. The training pant has a pair of laterally opposite side edges and a pair of longitudinally opposite waist edges.

The illustrated pant 20 may include a chassis 32, a pair of laterally opposite front side panels 34 extending laterally outward at the front region 22 and a pair of laterally opposite back side panels 134 extending laterally outward at the back region 24.

Referring to FIGs 1 and 2, the chassis 32 includes a backsheet 40 and a topsheet 42 that may be joined to the backsheet 40 in a superimposed relation therewith by adhesives, ultrasonic bonds, thermal bonds or other conventional techniques. The chassis 32 may further include an absorbent core 44 such as shown in FIG 2 disposed between the backsheet 40 and the topsheet 42 for absorbing fluid body exudates exuded by the wearer, and may further include a pair of containment flaps 46 secured to the topsheet 42 or the absorbent core 44 for inhibiting the lateral flow of body exudates.

The backsheet 40, the topsheet 42 and the absorbent core 44 may be made from many different materials known to those skilled in the art. All three layers, for instance, may be extensible and/or elastically extensible. Further, the stretch properties of each layer may vary in order to control the overall stretch properties of the product.

The backsheet 40, for instance, may be breathable and/or may be fluid impermeable. The backsheet 40 may be constructed of a single layer, multiple layers, laminates, spunbond fabrics, films, meltblown fabrics, elastic netting, microporous webs or bonded-carded-webs. The backsheet 40, for instance, can be a single layer of a fluid impermeable material, or alternatively can be a multi-layered laminate structure in which at least one of the layers is fluid impermeable.

The backsheet 40 can be biaxially extensible and optionally biaxially elastic. Elastic non-woven laminate webs that can be used as the backsheet 40 include a non-woven material joined to one or more gatherable non-woven webs or films. Stretch Bonded Laminates (SBL) and Neck Bonded Laminates (NBL) are examples of elastomeric composites.

Examples of suitable nonwoven materials are spunbond-meltblown fabrics, spunbond-meltblown-spunbond fabrics, spunbond fabrics, or laminates of such fabrics with films, or other nonwoven webs. Elastomeric materials may include cast or blown films, meltblown fabrics or spunbond fabrics composed of polyethylene, polypropylene, or polyolefin elastomers, as well as combinations thereof. The elastomeric materials may include PEBAX elastomer (available from AtoFina Chemicals, Inc., a business having offices located in Philadelphia, Pennsylvania U.S.A), HYTREL elastomeric polyester (available from Invista, a business having offices located in Wichita, Kansas U.S.A.), KRATON elastomer (available from Kraton Polymers, a business having offices located in Houston, Texas, U.S.A.), or strands of LYCRA elastomer (available from Invista), or the like, as well as combinations thereof. The backsheet 40 may include materials that have elastomeric properties through a mechanical process, printing process, heating process or chemical treatment. For example, such materials may be apertured, creped, neck-stretched, heat activated, embossed, and micro-strained, and may be in the form of films, webs, and laminates.

One example of a suitable material for a biaxially stretchable backsheet 40 is a breathable elastic film/nonwoven laminate, such as described in U.S. Patent 5,883,028, to Morman et al. Examples of materials having two-way stretchability and retractability are disclosed in U.S. Patents 5,116,662 to Morman and 5,114,781 to Morman. These two patents describe composite elastic materials capable of stretching in at least two directions. The materials have at least one elastic sheet and at least one necked material, or reversibly necked material, joined to the elastic sheet at least at three locations arranged in a nonlinear configuration, so that the necked, or reversibly necked, web is gathered between at least two of those locations.

The topsheet 42 is suitably compliant, soft-feeling and non-irritating to the wearer's skin. The topsheet 42 is also sufficiently liquid permeable to permit liquid body exudates to readily penetrate through its thickness to the absorbent core 44. A suitable topsheet 42 may be manufactured from a wide selection of web materials, such as porous foams, reticulated foams, apertured plastic films, woven and non-woven webs, or a combination of any such materials. For example, the topsheet 42 may include a meltblown web, a spunbonded web, or a bonded-carded-web composed of natural fibers, synthetic fibers or combinations thereof. The topsheet 42 may be composed of a substantially hydrophobic material, and the hydrophobic material may optionally be treated with a surfactant or otherwise processed to impart a desired level of wettability and hydrophilicity.

The topsheet 42 may also be extensible and/or elastomerically extensible. Suitable elastomeric materials for construction of the topsheet 42 can include elastic strands, LYCRA elastics, cast or blown elastic films, nonwoven elastic webs, meltblown or spunbond elastomeric fibrous webs, as well as combinations thereof. Examples of suitable elastomeric materials include KRATON elastomers, HYTREL elastomers, ESTANE elastomeric polyurethanes (available from Noveon, a business having offices located in Cleveland, Ohio U.S.A.), or PEBAX elastomers. The topsheet 42 can also be made from extensible materials such as those described in U.S. Patent 6,552,245 to Roessler et al. The topsheet 42 can also be made from biaxially stretchable materials as described in U.S. Patent 6,641,134 filed to Vukos et al.

The article 20 can optionally further include a surge management layer which may be located adjacent the absorbent core 44 and attached to various components in the article 20 such as the absorbent core 44 or the topsheet 42 by methods known in the art, such as by using an adhesive. In general, a surge management layer helps to quickly acquire and diffuse surges or gushes of liquid that may be rapidly introduced into the absorbent structure of the article. The surge management layer can temporarily store the liquid prior to releasing it into the storage or retention portions of the absorbent core 44. Examples of suitable surge management layers are described in U.S. Patents 5,486,166 to Bishop et al.; 5,490,846 to Ellis et al.; and 5,820,973 to Dodge et al.

The article 20 can further comprise an absorbent core 44. The absorbent core 44 may have any of a number of shapes. For example, it may have a 2-dimensional or 3-dimensional configuration, and may be rectangular shaped, triangular shaped, oval shaped, race-track shaped, I-shaped, generally hourglass shaped, T-shaped and the like. It is often suitable for the absorbent core 44 to be narrower in the crotch portion 26 than in the rear 24 or front 22 portion(s). The absorbent core 44 can be attached in an absorbent article, such as to the backsheet 40 and/or the topsheet 42 for example, by bonding means known in the art, such as ultrasonic, pressure, adhesive, aperturing, heat, sewing thread or strand, autogenous or self-adhering, hook-and-loop, or any combination thereof.

In some aspects, the absorbent core 44 can have a significant amount of stretchability. For example, the absorbent core 44 can comprise a matrix of fibers which includes an operative amount of elastomeric polymer fibers. Other methods known in the art can include attaching superabsorbent materials to a stretchable film, utilizing a nonwoven substrate having cuts or slits in its structure, and the like.

The absorbent core 44 can also include absorbent material, such as superabsorbent material and/or fluff. Additionally, the superabsorbent material can be operatively contained within a matrix of fibers, such as polymeric fibers. Accordingly, the absorbent core 44 can comprise a quantity of superabsorbent material and/or fluff contained within a matrix of fibers. In some aspects, the amount of superabsorbent material in the absorbent core 44 can be at least about 10% by weight of the core, such as at least about 30%, or at least about 60% by weight or at least about 80%, or between about 10% and about 80% by weight of the core to provide improved benefits. Optionally, the amount of superabsorbent material can be at least about 95-percent by weight of the core. In other aspects, the absorbent core 44 can comprise about 35-percent or less by weight fluff, such as about 25-percent or less, or 15-percent or less by weight fluff.

It should be understood that the present invention is not restricted to use with superabsorbent materials and/or fluff. In some aspects, the absorbent core 44 may additionally or alternatively include materials such as surfactants, ion exchange resin particles, moisturizers, emollients, perfumes, natural fibers, synthetic fibers, fluid modifiers, odor control additives, and combinations thereof. Alternatively, the absorbent core 44 can be or can include a foam.

In order to function well, the absorbent core 44 can have certain desired properties to provide improved performance as well as greater comfort and confidence among the user. For instance, the absorbent core 44 can have corresponding configurations of absorbent capacities, densities, basis weights and/or sizes which are selectively constructed and arranged to provide desired combinations of absorbency properties such as liquid intake rate, absorbent capacity, liquid distribution or fit properties such as shape maintenance and aesthetics. Likewise, the components can have desired wet to dry strength ratios, mean flow pore sizes, permeabilities and elongation values.

As mentioned above, the absorbent core 44 can optionally include elastomeric polymer fibers. The elastomeric material of the polymer fibers may include an olefin elastomer or a non-olefin elastomer, as desired. For example, the elastomeric fibers can include olefinic copolymers, polyethylene elastomers, polypropylene elastomers, polyester elastomers, polyisoprene, cross-linked polybutadiene, diblock, triblock, tetrablock, or other multi-block thermoplastic elastomeric and/or flexible copolymers such as block copolymers including hydrogenated butadiene-isoprene-butadiene block copolymers; stereoblock polypropylenes; graft copolymers, including ethylene-propylene-diene terpolymer or ethylene-propylene-diene monomer (EPDM) rubber, ethylene-propylene random copolymers (EPM), ethylene propylene rubbers (EPR), ethylene vinyl acetate (EVA), and ethylene-methyl acrylate (EMA); and styrenic block copolymers including diblock and triblock copolymers such as styrene-isoprene-styrene (SIS), styrene-butadiene-styrene (SBS), styrene-isoprene-butadiene-styrene (SIBS), styrene-ethylene/butylene-styrene (SEBS), or styrene-ethylene/propylene-styrene (SEPS), which may be obtained from Kraton Inc. under the trade designation KRATON elastomeric resin or from Dexco, a division of ExxonMobil Chemical Company (a business having offices located in Houston, Texas U.S.A.) under the trade designation VECTOR (SIS and SBS polymers); blends of thermoplastic elastomers with dynamic vulcanized elastomer-thermoplastic blends; thermoplastic polyether ester elastomers; ionomeric thermoplastic elastomers; thermoplastic elastic polyurethanes, including those available from Invista Corporation under the trade name LYCRA polyurethane, and ESTANE available from Noveon, Inc. (a business having offices located in Cleveland, Ohio U.S.A.); thermoplastic elastic polyamides, including polyether block amides available from AtoFina Chemicals, Inc. (a business having offices located in Philadelphia, Pennsylvania U.S.A). under the trade name PEBAX; polyether block amide; thermoplastic elastic polyesters, including those available from E. I. Du Pont de Nemours Co., under the trade name HYTREL, and ARNITEL from DSM Engineering Plastics (a business having offices located in Evansville, Indiana, U.S.A.) and single-site or metallocene-catalyzed polyolefins having a density of less than about 0.89 grams/cubic centimeter, available from Dow Chemical Co. (a business having offices located in Freeport, Texas U.S.A.) under the trade name AFFINITY; and combinations thereof.

As used herein, a tri-block copolymer has an ABA structure where the A represents several repeat units of type A, and B represents several repeat units of type B. As mentioned above, several examples of styrenic block copolymers are SBS, SIS, SIBS, SEBS and SEPS. In these copolymers the A blocks are polystyrene and the B blocks are a rubbery component. Generally, these triblock copolymers have molecular weights that can vary from the low thousands to hundreds of thousands, and the styrene content can range from 5% to 75% based on the weight of the triblock copolymer. A diblock copolymer is similar to the triblock, but is of an AB structure. Suitable diblocks include styrene-isoprene diblocks, which have a molecular weight of approximately one-half of the triblock molecular weight having the same ratio of A blocks to B blocks.

In desired arrangements, the polymer fibers can include at least one material selected from the group consisting of styrenic block copolymers, elastic polyolefin polymers and co-polymers and EVA/EMA type polymers.

In other particular arrangements, for example, the elastomeric material of the polymer fibers can include various commercial grades of low crystallinity, lower molecular weight metallocene polyolefins, available from ExxonMobil Chemical Company (a company having offices located in Houston, Texas, U.S.A.) under the VISTAMAXX trade designation. The VISTAMAXX material is believed to be metallocene propylene ethylene copolymer. For example, in one aspect the elastomeric polymer can be VISTAMAXX PLTD 2210. In other aspects, the elastomeric polymer can be VISTAMAXX PLTD 1778. Another optional elastomeric polymer is KRATON blend G 2755 from Kraton Inc. The KRATON material is believed to be a blend of styrene ethylene-butylene styrene polymer, ethylene waxes and tackifying resins.

In some aspects, the elastomeric polymer fibers can be produced from a polymer material having a selected melt flow rate (MFR). In a particular aspect, the MFR can be up to a maximum of about 300. Alternatively, the MFR can be up to about 230 or 250. In another aspect, the MFR can be a minimum of not less than about 20. The MFR can alternatively be not less than about 50 to provide desired performance. The described melt flow rate has the units of grams flow per 10 minutes (g/10 min). The parameter of melt flow rate is well known, and can be determined by conventional techniques, such as by employing test ASTM D 1238 70 "extrusion plastometer" Standard Condition "L" at 230°C and 2.16 kg applied force.

As mentioned above, the polymer fibers of the absorbent core 44 can include an amount of a surfactant. The surfactant can be combined with the polymer fibers of the absorbent core in any operative manner. Various techniques for combining the surfactant are conventional and well known to persons skilled in the art. For example, the surfactant may be compounded with the polymer employed to form a meltblown fiber structure. In a particular feature, the surfactant may be configured to operatively migrate or segregate to the outer surface of the fibers upon the cooling of the fibers. Alternatively, the surfactant may be applied to or otherwise combined with the polymer fibers after the fibers have been formed.

The polymer fibers can include an operative amount of surfactant, based on the total weight of the fibers and surfactant. In some aspects, the polymer fibers can include at least a minimum of about 0.1 % by weight surfactant, as determined by water extraction. The amount of surfactant can alternatively be at least about 0.15% by weight, and can optionally be at least about 0.2% by weight to provide desired benefits. In other aspects, the amount of surfactant can be generally not more than a maximum of about 2% by weight, such as not more than about 1% by weight, or not more than about 0.5% by weight to provide improved performance.

If the amount of surfactant is outside the desired ranges, various disadvantages can occur. For example, an excessively low amount of surfactant may not allow fibers, such as hydrophobic meltblown fibers, to wet with the absorbed fluid. In contrast, an excessively high amount of surfactant may allow the surfactant to wash off from the fibers and undesirably interfere with the ability of the absorbent core to transport fluid, or may adversely affect the attachment strength of the absorbent core 44 to the absorbent article 20. Where the surfactant is compounded or otherwise internally added to the polymer fibers, an excessively high level of surfactant can create conditions that cause a poor formation of the polymer fibers.

In some configurations, the surfactant can include at least one material selected from the group that includes polyethylene glycol ester condensates and alkyl glycoside surfactants. For example, the surfactant can be a GLUCOPON surfactant, available from Cognis Corporation (a business having offices located in Cincinnati, Ohio, U.S.A.) which can be composed of 40-percent water, and 60-percent D-glucose, decyl, octyl ethers and oligomerics.

In other aspects of the invention, the surfactant can be in the form of a sprayed-on surfactant comprising a water/surfactant solution which includes 16 liters of hot water (about 45 °C to 50 °C) mixed with 0.20 kg of GLUCOPON 220 UP surfactant available from Cognis Corporation and 0.36 kg of AHCHOVEL Base N-62 surfactant available from Uniqema (a business having offices located in New Castle, Delaware, U.S.A.). When employing a sprayed-on surfactant, a relatively lower amount of sprayed-on surfactant may be desirable to provide the desired containment of the superabsorbent material. Excessive amounts of the fluid surfactant may hinder the desired attachment of the superabsorbent material to the molten, elastomeric meltblown fibers, for example.

An example of an internal surfactant or wetting agent that can be compounded with the elastomeric fiber polymer can include a MAPEG DO 400 PEG (polyethylene glycol) ester, available from BASF (a business having offices located in Freeport, Texas, U.S.A.). Other internal surfactants can include a polyether, a fatty acid ester, a soap or the like, as well as combinations thereof.

The absorbent core 44 also includes a desired amount of superabsorbent material of the present invention. Superabsorbent materials typically are polymers of unsaturated carboxylic acids or derivatives thereof. These polymers are rendered water insoluble, but water swellable, by crosslinking the polymer with a di- or poly-functional internal crosslinking agent. These internally crosslinked polymers are at least partially neutralized and contain pendant anionic carboxyl groups on the polymer backbone that enable the polymer to absorb aqueous fluids, such as body fluids.

Superabsorbent materials are manufactured by known polymerization techniques, preferably by polymerization in aqueous solution by gel polymerization. The products of this polymerization process are aqueous polymer gels (i.e., superabsorbent hydrogels) that are reduced in size to small particles by mechanical forces, then dried using drying procedures and apparatus known in the art. The drying process is followed by pulverization of the resulting superabsorbent material to the desired particle size.

To improve the fluid absorption profile, superabsorbent materials are optimized with respect to one or more of absorption capacity, absorption rate, acquisition time, gel strength, and/or permeability. Optimization allows for a reduction in the amount of fluff fiber used in an absorbent article, which results in a thinner article. However, it is very difficult to maximize all of these absorption profile properties simultaneously. Nonetheless, the superabsorbent materials of the present invention maintain the conflicting properties of a high centrifuge retention capacity (CRC) and an excellent permeability, and can provide for an absorbent article with improved fluid intake rate.

One method of optimizing the fluid absorption profile of superabsorbent materials is to provide superabsorbent materials of a predetermined particle size distribution. In particular, particles that are too small in size tend to swell after absorbing a fluid and can block the absorption of further fluid. Particles that are too large in size have a reduced surface area which decreases the rate of absorption.

Therefore, the particle size distribution of the superabsorbent materials should be such that fluid permeability, absorption, and retention by the superabsorbent materials are maximized. Any subsequent process that agglomerates the superabsorbent materials to provide oversized particles should be avoided. In particular, agglomeration of superabsorbent materials increases apparent particle size, which reduces the surface area of the superabsorbent materials, and in turn adversely affects absorption of an aqueous fluid by the superabsorbent materials.

The superabsorbent materials of the present invention maintain the conflicting properties of a high centrifuge retention capacity (CRC) and an excellent permeability. These problems are overcome in part because of a polyammonium carbonate coating, and in part because of a reduced tendency of the present coated superabsorbent materials to agglomerate.

In order to use an increased amount of superabsorbent materials and a decreased amount of fluff in the absorbent core 44, it is important to maintain a high liquid permeability. In particular, the permeability of a superabsorbent hydrogel layer formed by swelling in the presence of a fluid is very important to overcome the problem of leakage from the product. A lack of permeability directly impacts the ability of superabsorbent hydrogel layers to acquire and distribute such fluids.

In general, the coating of superabsorbent materials with uncrosslinked polyamines can improve adhesion to cellulosic fluff fibers because of the high flexibility of polyamine molecules. However, low molecular weight, uncrosslinked polyamines can be extracted from the superabsorbent materials by wetting with an aqueous fluid. As a result, the viscosity of the aqueous fluid increases, and the acquisition rate of the superabsorbent materials is reduced. If a polyamine is covalently bound to the superabsorbent materials, the degree of superabsorbent material crosslinking is increased and the absorptive capacity of the material is undesirably reduced. Moreover, covalent bonding of polyamine to a superabsorbent material surface typically occurs at a temperature greater than 150°C, which adversely affects the color of the superabsorbent materials and, ultimately, consumer acceptance of the absorbent article.

In accordance with the present invention, optionally surface-crosslinked superabsorbent materials can be coated with a polyamine via a polyammonium carbonate precursor. The present superabsorbent materials comprise a base polymer which can be a homopolymer or a copolymer. The identity of the base polymer is not limited as long as the polymer is an anionic polymer (i.e., contains pendant acid moieties) and is capable of swelling and absorbing at least ten times its weight in water when in a neutralized form. Suitable base polymers are crosslinked polymers having acid groups that are at least partially in the form of a salt, generally an alkali metal or ammonium salt.

The base polymer typically has at least about 25 mol% of the pendant acid moieties, (e.g., carboxylic acid moieties) present in a neutralized form, such as between about 25 mol% and about 100 mol%, or between about 50 mol% and about 100 mol% of the pendant acid moieties present in a neutralized form. In accordance with the present invention, the base polymer preferably has a Degree of Neutralization (DN) between about 25 mol% and 100 mol%.

The base polymer of the superabsorbent material of the present invention is a lightly crosslinked polymer capable of absorbing several times its own weight in water and/or saline. Superabsorbent materials can be made by any conventional process for preparing superabsorbent polymers and are well known to those skilled in the art. One process for preparing superabsorbent materials is a solution polymerization method described in U.S. Patent Nos. 4,076,663; 4,286,082; 4,654,039; and 5,145,906. Another process is an inverse suspension polymerization method described in U.S. Patent Nos. 4,340,706; 4,497,930; 4,666,975; 4,507,438; and 4,683,274.

Superabsorbent materials suitable in the present invention may be prepared from one or more monoethylenically unsaturated compounds having at least one acid moiety, such as carboxyl, carboxylic acid anhydride, carboxylic acid salt, sulfonic acid, sulfonic acid salt, sulfuric acid, sulfuric acid salt, phosphoric acid, phosphoric acid salt, phosphonic acid, or phosphonic acid salt. Superabsorbent materials useful in the present invention suitably are prepared from one or more monoethylenically unsaturated, water-soluble carboxyl or carboxylic acid anhydride containing monomer, and the alkali metal and ammonium salts thereof, wherein these monomers desirably comprise 50 mol% to 99.9 mol% of the base polymer.

The base polymer of the superabsorbent materials of the present invention is suitably a lightly crosslinked acrylic resin, such as lightly crosslinked polyacrylic acid. The lightly crosslinked base polymer can be prepared by polymerizing an acidic monomer containing an acyl moiety, such as acrylic acid, or a moiety capable of providing an acid group (e.g., acrylonitrile) in the presence of an internal crosslinking agent (e.g., a polyfunctional organic compound). The base polymer can contain other copolymerizable units (e.g., other monoethylenically unsaturated comonomers known in the art) as long as the base polymer is at least about 10 mol%, such as at least about 25 mol%, acidic monomer units, such as (meth)acrylic acid. To achieve the full advantage of the present invention, the base polymer preferably contains at least about 50 mol%, such as at least about 75 mol%, or up to about 100 mol%, acidic monomer units. The other copolymerizable units can, for example, help improve the hydrophilicity of the polymer.

Suitable ethylenically unsaturated carboxylic acid and carboxylic acid anhydride monomers useful in the base polymer include acrylic acid, methacrylic acid, ethacrylic acid, α-chloroacrylic acid, α-cyanoacrylic acid, β-methylacrylic acid (crotonic acid), α-phenylacrylic acid, β-acryloxypropionic acid, sorbic acid, α-chlorosorbic acid, angelic acid, cinnamic acid, p-chlorocinnamic acid, β-stearylacrylic acid, itaconic acid, citraconic acid, mesaconic acid, glutaconic acid, aconitic acid, maleic acid, fumaric acid, tricarboxyethylene, and maleic anhydride.

Suitable ethylenically unsaturated sulfonic and phosphonic acid monomers include aliphatic or aromatic vinyl sulfonic acids, such as vinylsulfonic acid, allyl sulfonic acid, vinyl toluene sulfonic acid, styrene sulfonic acid, acrylic and methacrylic sulfonic acids (e.g., sulfoethyl acrylate, sulfoethyl methacrylate, sulfopropyl acrylate, sulfopropyl methacrylate, 2-hydroxy-3-methacryloxypropyl sulfonic acid, and 2-acrylamido-2-methylpropane sulfonic acid); vinylphosphonic acid; allylphosphonic acid; and mixtures thereof.

Exemplary, but nonlimiting, monomers include acrylic acid, methacrylic acid, maleic acid, fumaric acid, maleic anhydride, and the sodium, potassium, and ammonium salts thereof. One particularly suitable monomer is acrylic acid.

The base polymer can also contain additional monoethylenically unsaturated monomers that do not bear a pendant acid group, but are copolymerizable with monomers bearing acid groups. Such compounds include, for example, the amides and nitriles of monoethylenically unsaturated carboxylic acids, including acrylamide, methacrylamide, acrylonitrile, and methacrylonitrile. Examples of other suitable comonomers include, but are not limited to, vinyl esters of saturated C₁₋₄ carboxylic acids, such as vinyl formate, vinyl acetate, and vinyl propionate; alkyl vinyl ethers having at least two carbon atoms in the alkyl group, for example, ethyl vinyl ether and butyl vinyl ether; esters of monoethylenically unsaturated C₃₋₁₈ alcohols and acrylic acid, methacrylic acid, or maleic acid; monoesters of maleic acid, for example, methyl hydrogen maleate; acrylic and methacrylic esters of alkoxylated monohydric saturated alcohols, for example, alcohols having 10 to 25 carbon atoms reacted with 2 to 200 moles of ethylene oxide and/or propylene oxide per mole of alcohol; and monoacrylic esters and monomethacrylic esters of polyethylene glycol or polypropylene glycol, the molar masses (Mₙ) of the polyalkylene glycols being up to about 2,000, for example. Further suitable comonomers include, but are not limited to, styrene and alkyl-substituted styrenes, such as ethylstyrene and tert-butylstyrene, and 2-hydroxyethyl acrylate.

Polymerization of the acidic monomers, and any copolymerizable monomers, can be performed by free radical processes in the presence of a polyfunctional organic compound. The base polymers are internally crosslinked to a sufficient extent such that the base polymer is water insoluble. Internal crosslinking renders the base polymer substantially water insoluble and, in part, serves to determine the absorption capacity of the base polymer. For use in absorption applications, the base polymer is suitably lightly crosslinked, having a crosslinking density of less than about 20%, such as less than about 10%, or between about 0.01% to about 7%.

A crosslinking agent is suitably used in an amount of less than about 7 wt%, such as between about 0.1 wt% and about 5 wt%, based on the total weight of monomers. Examples of crosslinking polyvinyl monomers include, but are not limited to, polyacrylic (or polymethacrylic) acid esters represented by the following formula (I), and bisacrylamides represented by the following formula (II): 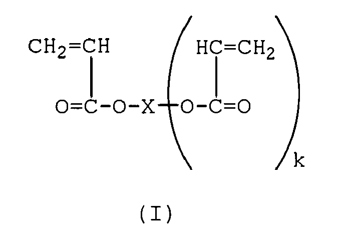 wherein X is ethylene, propylene, trimethylene, cyclohexyl, hexamethylene, 2-hydroxypropylene, (CH₂CH₂O)ₙCH₂CH₂, or 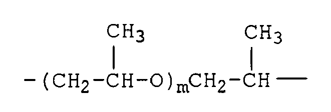 n and m are each an integer 5 to 40, and k is 1 or 2; 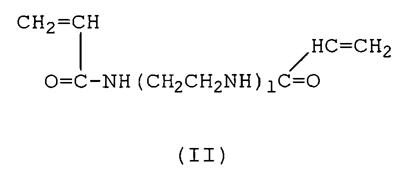 wherein I is 2 or 3.

The compounds of formula (I) can be prepared by reacting polyols, such as ethylene glycol, propylene glycol, trimethylolpropane, 1,6-hexanediol, glycerin, pentaerythritol, polyethylene glycol, or polypropylene glycol, with acrylic acid or methacrylic acid. The compounds of formula (II) can be obtained by reacting polyalkylene polyamines, such as diethylenetriamine and triethylenetetramine, with acrylic acid.

Specific internal crosslinking agents include, but are not limited to, 1,4-butanediol diacrylate, 1,4-butanediol dimethacrylate, 1,3-butylene glycol diacrylate, 1,3-butylene glycol dimethacrylate, diethylene glycol diacrylate, diethylene glycol dimethacrylate, ethoxylated bisphenol A diacrylate, ethoxylated bisphenol A dimethacrylate, ethylene glycol dimethacrylate, 1,6-hexanediol diacrylate, 1,6-hexanediol dimethacrylate, neopentyl glycol dimethacrylate, polyethylene glycol diacrylate, polyethylene glycol dimethacrylate, triethylene glycol diacrylate, triethylene glycol dimethacrylate, tripropylene glycol diacrylate, tetraethylene glycol diacrylate, tetraethylene glycol dimethacrylate, dipentaerythritol pentaacrylate, pentaerythritol tetraacrylate, pentaerythritol triacrylate, trimethylolpropane triacrylate, trimethylolpropane trimethacrylate, tris(2-hydroxyethyl)-isocyanurate triacrylate, ethoxylated trimethylolpropane triacrylate (ETMPTA) (e.g., ETMPTA ethyoxylated with 15 moles of ethylene oxide (EO) on average), tris(2-hydroxyethyl)isocyanurate trimethyacrylate, divinyl esters of a polycarboxylic acid, diallyl esters of a polycarboxylic acid, triallyl terephthalate, diallyl maleate, diallyl fumarate, hexamethylenebismaleimide, trivinyl trimellitate, divinyl adipate, diallyl succinate, a divinyl ether of ethylene glycol, cyclopentadiene diacrylate, a tetraallyl ammonium halide, divinyl benzene, divinyl ether, diallyl phthalate, or mixtures thereof. Particularly suitable internal crosslinking agents are N,N'-methylenebisacrylamide, N,N'-methylenebismethacrylamide, ethylene glycol dimethacrylate, and trimethylolpropane triacrylate.

The base polymer of the present invention can be any internally crosslinked polymer having pendant acid moieties that acts as a superabsorbent material in its neutralized form. Examples of suitable base polymers include, but are not limited to, polyacrylic acid, hydrolyzed starch-acrylonitrile graft copolymers, starch-acrylic acid graft copolymers, saponified vinyl acetate-acrylic ester copolymers, hydrolyzed acrylonitrile copolymers, hydrolyzed acrylamide copolymers, ethylene-maleic anhydride copolymers, isobutylene-maleic anhydride copolymers, poly(vinylsulfonic acid), poly(vinylphosphonic acid), poly(vinylphosphoric acid), poly(vinylsulfuric acid), sulfonated polystyrene, poly(aspartic acid), poly(lactic acid), and mixtures thereof. The preferred base polymer is a homopolymer or copolymer of acrylic acid or methacrylic acid.

The free radical polymerization can be initiated by an initiator or by electron beams acting on a polymerizable aqueous mixture. Polymerization also can be initiated in the absence of such initiators by the action of high energy radiation in the presence of photoinitiators.

Useful polymerization initiators include, but are not limited to, compounds that decompose into free radicals under polymerization conditions, for example, peroxides, hydroperoxides, persulfates, azo compounds, and redox catalysts. Water-soluble initiators are desirable. In some cases, mixtures of different polymerization initiators are used, such as, mixtures of hydrogen peroxide and sodium peroxodisulfate or potassium peroxodisulfate, for example. Mixtures of hydrogen peroxide and sodium peroxodisulfate can be in any proportion.

Examples of suitable organic peroxides include, but are not limited to, acetylacetone peroxide, methyl ethyl ketone peroxide, tert-butyl hydroperoxide, cumene hydroperoxide, tert-amyl perpivalate, tert-butyl perpivalate, tert-butyl perneohexanoate, tert-butyl perisobutyrate, tert-butyl per-2-ethylhexanoate, tert-butyl perisononanoate, tert-butyl permaleate, tert-butyl perbenzoate, di(2-ethylhexyl) peroxydicarbonate, dicyclohexyl peroxydicarbonate, di(4-tert-butylcyclohexyl) peroxydicarbonate, dimyristyl peroxydicarbonate, diacetyl peroxydicarbonate, an allyl perester, cumyl peroxyneodecanoate, tert-butyl per-3,5,5-trimethylhexanoate, acetylcyclohexylsulfonyl peroxide, dilauryl peroxide, dibenzoyl peroxide, and tert-amyl pemeodecanoate. Particularly suitable polymerization initiators are water-soluble azo initiators, for example 2,2'-azobis(2-amidinopropane) dihydrochloride, 2,2'-azobis(N,N'-dimethylene)isobutyramidine dihydrochloride, 2-(carbamoylazo-isobutyronitrile, 2,2'-azobis[2-(2'-imidazolin-2-yl)propane] dihydrochloride, and 4,4'-azobis(4-cyanovaleric acid). The polymerization initiators are used, for example, in amounts of between about 0.01% to about 5%, such as about 0.05% to about 2.0% by weight, based on the monomers to be polymerized.

Suitable polymerization initiators also include redox catalysts. In redox catalysts, the oxidizing compound comprises at least one of the above-specified per compounds, and the reducing component comprises, for example, ascorbic acid, glucose, sorbose, ammonium or alkali metal bisulfite, sulfite, thiosulfate, hyposulfite, pyrosulfite, or sulfide, or a metal salt, such as iron (II) ions or sodium hydroxymethylsulfoxylate. The reducing component of the redox catalyst preferably is ascorbic acid or sodium sulfite. Based on the amount of monomers used in the polymerization, about 3 x 10⁻⁶ to about 1 mol% of the reducing component of the redox catalyst system can be used, and about 0.001 to about 5.0 mol% of the oxidizing component of the redox catalyst can be used, for example.

When polymerization is initiated using high energy radiation, the initiator typically comprises a photoinitiator. Photoinitiators include, for example, α-splitters, H-abstracting systems, and azides. Examples of such initiators include, but are not limited to, benzophenone derivatives, such as Michler's ketone; phenanthrene derivatives; fluorene derivatives; anthraquinone derivatives; thioxanthone derivatives; coumarin derivatives; benzoin ethers and derivatives thereof; azo compounds, such as the abovementioned free-radical formers, substituted hexaarylbisimidazoles, acylphosphine oxides; or mixtures thereof.

Examples of azides include, but are not limited to, 2-(N,N-dimethylamino)ethyl 4-azidocinnamate, 2-(N,N-dimethylamino)ethyl 4-azidonaphthyl ketone, 2-(N,N-dimethylamino)ethyl 4-azidobenzoate, 5-azido-1-naphthyl 2'-(N,N-dimethylamino)ethyl sulfone, N-(4-sulfonylazidophenyl)maleimide, N-acetyl-4-sulfonylazidoaniline, 4-sulfonyl-azidoaniline, 4-azidoaniline, 4-azidophenacyl bromide, p-azidobenzoic acid, 2,6-bis(p-azidobenzylidene)cyclohexanone, and 2,6-bis(p-azidobenzylidene)-4-methylcyclohexanone. Photoinitiators can be used, if at all, in amounts of about 0.01 % to about 5% by weight of the monomers to be polymerized.

As previously stated, the base polymer is partially neutralized. The degree of neutralization preferably is greater than about 25 mol%, such as between about 50 mol% and about 100 mol%, or between about 70 mol% and about 100 mol%, based on monomers containing acid groups.

Useful neutralizing agents for the base polymer include alkali metal bases, ammonia, and/or amines. Preferably, the neutralizing agent comprises aqueous sodium hydroxide, aqueous potassium hydroxide, or lithium hydroxide. However, neutralization also can be achieved using sodium carbonate, sodium bicarbonate, potassium carbonate, or potassium bicarbonate, or other carbonates or bicarbonates, as a solid or as a solution. Primary, secondary, and/or tertiary amines can also be used to neutralize the base polymer.

Neutralization of the base polymer can be performed before, during, or after the polymerization in a suitable apparatus for this purpose. For example, the neutralization can be performed directly in a kneader used for polymerization of the monomers.

In accordance with the present invention, polymerization of an aqueous monomer solution (e.g., gel polymerization) is desirable. In this method, a 10% to 70% by weight aqueous solution of the monomers, including the internal crosslinking agent, is neutralized in the presence of a free radical initiator. The solution polymerization is suitably performed at about 0°C to about 150°C, such as about 10°C to about 100°C, and at atmospheric, superatmospheric, or reduced pressure. The polymerization also can be conducted under a protective gas atmosphere, suitably under nitrogen.

After polymerization, the resulting hydrogel of the base polymer is dried, and the dry base polymer materials are ground and classified. In accordance with the present invention, surface crosslinking is optional. However, the base polymer particles typically are indeed surface cross-linked. The base polymer particles can first be surface crosslinked, then coated with a polyammonium carbonate and heated to provide a polyamine coating. Suitably, surface crosslinking is performed simultaneously with applying a polyammonium carbonate on the base polymer particles.

Polyamines are viscous compounds, and typically are dissolved in a suitable solvent to facilitate handling and allow facile application onto the surfaces of superabsorbent materials. These solutions also may be viscous, which often make it difficult to homogeneously apply a polyamine to superabsorbent material surfaces. To overcome this problem, a dilute solution of a polyamine can be used. However, this additional amount of solvent then must be evaporated from the superabsorbent materials, which increases production time and is costly.

In accordance with the present invention, a polyamine is converted into a polyammonium carbonate by passing carbon dioxide through a polyamine solution. The polyammonium carbonate precipitates from the solution to provide a slurry or dispersion of the precipitated polyammonium carbonate. The polyammonium carbonate slurry has a reduced viscosity compared to a polyamine solution, and does not impart a tack to surfaces of the superabsorbent materials.

Therefore, a polyammonium carbonate first is prepared. In one aspect of preparing polyamine-coated base polymer particles, a coating solution containing the polyammonium carbonate dispersed in a solvent is applied to the surfaces of the base polymer particles. Then, coating solution(s) containing an optional inorganic salt having a polyvalent metal cation and/or an optional surface crosslinking agent dissolved or dispersed in a suitable solvent can be applied to the surfaces of the superabsorbent materials. Then, the coated base polymer particles are heated for a sufficient time at a sufficient temperature to evaporate the solvents of the coating solutions, to surface crosslink the base polymer particles (if an optional surface crosslinking agent is used), and to form a polyamine coating on the base polymer particles to provide superabsorbent materials of the present invention.

It should be understood that the order of applying the polyammonium carbonate, optional inorganic salt, and optional surface crosslinking agent to the surfaces of the base polymer particles is not critical. The components can be added in any order, either from two or three solutions. However, the polyammonium carbonate and optional inorganic salt should be applied from different solutions to avoid an interaction prior to application to the base polymer particles.

In other aspects, the base polymer particles can be surface crosslinked prior to application of the polyammonium carbonate and optional inorganic salt. In still other aspects, a surface crosslinking agent can be applied to the base polymer particles, followed by the polyammonium carbonate and optional inorganic salt, and the particles are then heated to form surface crosslinks and the polyamine coating simultaneously.

In the optional surface crosslinking process, a multifunctional compound capable of reacting with the functional groups of the base polymer can be applied to the surface of the base polymer particles, preferably using an aqueous solution. The aqueous solution can also contain water-miscible organic solvents, such as an alcohol (e.g., such as methanol, ethanol, or i-propanol); a polyol, such as ethylene glycol or propylene glycol; or acetone.

A solution of a surface crosslinking agent is applied to the base polymer particles in an amount to wet predominantly only the outer surfaces of the base polymer particles, either before or after application of the polyamine. Surface crosslinking and drying of the base polymer particles can then be performed, suitably by heating at least the wetted surfaces of the base polymer particles.

Typically, the base polymer particles are surface treated with a solution of a surface crosslinking agent containing about 0.01 % to about 4% by weight surface crosslinking agent, such as about 0.4% to about 2% by weight surface crosslinking agent in a suitable solvent. The solution can be applied as a fine spray onto the surfaces of freely tumbling base polymer particles at a ratio of about 1:0.01 to about 1:0.5 parts by weight base polymer particles to solution of surface crosslinking agent. The surface crosslinking agent, if present at all, can be present in an amount of about 0.001 % to about 5% by weight of the base polymer particles, such as about 0.001 % to about 0.5% by weight. To achieve the full advantage of the present invention, the surface crosslinking agent is present in an amount of about 0.001 % to about 0.1% by weight of the base polymer particles.

Surface crosslinking and drying of the base polymer particles are achieved by heating the surface-treated base polymer particles at a suitable temperature, such as between about 70°C to about 150°C, or between about 105°C to about 120°C. Suitable surface crosslinking agents are capable of reacting with acid moieties and crosslinking polymers at the surfaces of the base polymer particles.

Nonlimiting examples of suitable surface crosslinking agents include, but are not limited to, an alkylene carbonate, such as ethylene carbonate or propylene carbonate; a polyaziridine, such as 2,2-bishydroxymethyl butanol tris[3-(1-aziridine propionate] or bis-N-aziridinomethane; a haloepoxy, such as epichlorhydrin; a polyisocyanate, such as 2,4-toluene diisocyanate; a dior polyglycidyl compound, such as diglycidyl phosphonates, ethylene glycol diglycidyl ether, or bischlorohydrin ethers of polyalkylene glycols; alkoxysilyl compounds; polyols such as ethylene glycol, 1,2-propanediol, 1,4-butanediol, glycerol, methyltriglycol, polyethylene glycols having an average molecular weight (M_{w}) of 200-10,000, di- and polyglycerol, pentaerythritol, sorbitol, the ethoxylates of these polyols and their esters with carboxylic acids or carbonic acid, such as ethylene carbonate or propylene carbonate; carbonic acid derivatives, such as urea, thiourea, guanidine, dicyandiamide, 2-oxazolidinone and its derivatives, bisoxazoline, polyoxazolines, di- and polyisocyanates; di- and poly-N-methylol compounds, such as methylenebis(N-methylolmethacrylamide) or melamine-formaldehyde resins; compounds having two or more blocked isocyanate groups, such as trimethylhexamethylene diisocyanate blocked with 2,2,3,6-tetramethylpiperidin-4-one; and other surface crosslinking agents known to persons skilled in the art.

A solution of the optional surface crosslinking agent can be applied to the surfaces of the base polymer particles before or after a solution containing the polyammonium carbonate is applied to the surfaces of the base polymer particles. The polyammonium carbonate also can be applied to the base polymer particles after the surface crosslinking step has been completed.

A dispersion or slurry containing the polyammonium carbonate preferably comprises about 5% to about 50% by weight of a polyammonium carbonate in a suitable solvent. The polyammonium carbonate can be prepared by passing a sufficient amount of carbon dioxide through a polyamine solution to convert the polyamine to a polyammonium carbonate and form a fine precipitate. Prior to passing carbon dioxide through a polyamine solution, the polyamine is dissolved in a sufficient amount of a solvent to allow the polyammonium carbonate to be readily and homogeneously applied to the surfaces of the base polymer particles. The solvent for the polyamine solution can be, but is not limited to, water, an alcohol, or a glycol, such as methanol, ethanol, ethylene glycol, or propylene glycol, and mixtures thereof.

The amount of polyammonium carbonate applied to the surfaces of the base polymer particles is suitably sufficient to coat the base polymer particle surfaces. Accordingly, the amount of polyammonium carbonate applied to the surfaces of the base polymer particles is preferably about 0.1 % to about 2%, such as about 0.2% to about 1 %, of the weight of the base polymer particle. To achieve the full advantage of the present invention, the polyammonium carbonate is present on the base polymer particle surfaces in an amount of about 0.2% to about 0.5% by weight of the base polymer particle.

The polyammonium carbonate applied to surfaces of the superabsorbent materials provides tack-free particles. Accordingly, the problem of superabsorbent material agglomeration is overcome. In addition, the viscosity of polyammonium carbonate dispersion is sufficiently low to permit a facile application of the dispersion onto superabsorbent material surfaces.

After heating the polyammonium carbonate-coated superabsorbent materials to drive off carbon dioxide and form a polyamine coating on superabsorbent material surfaces, the resulting polyamine forms an ionic bond with a base polymer and retains adhesive forces to the base polymer after the base polymer absorbs a fluid and swells. Desirably, an excessive amount of covalent bonds are not formed between the polyamine and the base polymer, and the polyamine-base polymer interactions are intermolecular, such as electrostatic, hydrogen bonding, and van der Waals interactions. Therefore, the presence of a polyamine on the base polymer particles does not adversely influence the absorption profile of the base polymer particles.

A polyamine useful in the present invention has at least two, and preferably a plurality of, nitrogen atoms per molecule. The polyamine typically has a weight average molecular weight (M_{w}) of about 5,000 to about 1,000,000, such as about 20,000 to about 300,000. To achieve the full advantage of the present invention, the polyamine has an M_{w} of about 100,000 to about 300,000.

In general, useful polyamine polymers have primary amine groups, secondary amine groups, tertiary amine groups, quaternary ammonium groups, or mixtures thereof. Examples of polyamines include, but are not limited to, a polyvinylamine, a polyallylamine, a polyethyleneimine, a polyalkyleneamine, a polyazetidine, a polyvinylguanidine, a poly(DADMAC) (i.e., a poly(diallyl dimethyl ammonium chloride), a cationic polyacrylamide, a polyamine functionalized polyacrylate, and mixtures thereof.

Homopolymers and copolymers of vinylamine also can be used, such as copolymers of vinylformamide and comonomers for example, which are converted to vinylamine copolymers. The comonomers can be any monomer capable of copolymerizing with vinylformamide. Nonlimiting examples of such monomers include, but are not limited to, acrylamide, methacrylamide, methacrylonitrile, vinylacetate, vinylpropionate, styrene, ethylene, propylene, N-vinylpyrrolidone, N-vinylcaprolactam, N-vinylimidazole, monomers containing a sulfonate or phosphonate group, vinylglycol, acrylamido(methacrylamido)alkylene trialkyl ammonium salt, diallyl dialkylammonium salt, C₁₋₄alkyl vinyl ethers such as methyl vinyl ether, ethyl vinyl ether, isopropyl vinyl ether, n-propyl vinyl ether, t-butyl vinyl ether, N-substituted alkyl (meth)acrylamides substituted by a C₁₋₄alkyl group as, for example, N-methylacrylamide, N-isopropylacrylamide, and N,N-dimethylacrylamide, C₁₋₂₀alkyl(meth)acrylic acid esters such as methyl methacrylate, ethyl methacrylate, propyl acrylate, butyl acrylate, hydroxyethyl acrylate, hydroxyethyl methacrylate, hydroxypropyl acrylate, hydroxypropyl methacrylate, hydroxybutyl acrylate, hydroxybutyl methacrylate, 2-methylbutyl acrylate, 3-methylbutyl acrylate, 3-pentyl acrylate, neopentyl acrylate, 2-methylpentyl acrylate, hexyl acrylate, cyclohexyl acrylate, 2-ethylhexyl acrylate, phenyl acrylate, heptyl acrylate, benzyl acrylate, tolyl acrylate, octyl acrylate, 2-octyl acrylate, nonyl acrylate, and octyl methacrylate.

Specific copolymers of polyvinylamine include, but are not limited to, copolymers of N-vinylformamide and vinyl acetate, vinyl propionate, a C₁₋₄alkyl vinyl ether, a (meth)acrylic acid ester, acrylonitrile, acrylamide, and vinylpyrrolidone.

In accordance with the present invention, the number of covalent bonds that form between the polyamine and the base polymer is low, if present at all. However, after heating the polyammonium carbonate-coated superabsorbent materials, the resulting polyamine may impart a tack to surfaces of the base polymer particles, which could lead to agglomeration or aggregation of coated base polymer particles. Therefore, a coating solution which contains an optional inorganic salt having a polyvalent cation having a valence of +2, +3, or +4 (i.e., in the range of +2 to +4) can be applied to the surfaces of the polyamine-coated base polymer particles.

The polyvalent metal cation is capable of interacting (e.g., forming ionic crosslinks) with the nitrogen atoms of the polyamine. As a result, a tackless, polyamine coating can be formed on the surface of the base polymer to provide coated superabsorbent materials of the present invention. These coated superabsorbent materials have a substantially reduced tendency to agglomerate.

In accordance with the present invention, an optional inorganic salt applied to surfaces of the base polymer particles has a sufficient water solubility such that polyvalent metal cations are available to interact with the nitrogen atoms of the polyamine. Accordingly, a useful inorganic salt has a water solubility of at least about 0.1 grams (g) of inorganic salt per 100 milliliters (ml) of water, such as at least about 0.2 g per 100 ml of water.

The polyvalent metal cation of the optional inorganic salt has a valence of +2, +3, or +4 (i.e., in the range of +2 to +4) and can be, but is not limited to, Mg²⁺, Ca²⁺, Al³⁺, Sc³⁺, Ti⁴⁺, Mn²⁺, Fe²⁺, Fe³⁺, Co²⁺, Ni²⁺, Cu^{+/2+}, Zn²⁺, Y³⁺, Zr⁴⁺, La³⁺, Ce⁴⁺, Hf⁴⁺, Au³⁺, and mixtures thereof. Desirable cations are Mg²⁺, Ca²⁺, Al³⁺, Ti⁴⁺, Zr⁴⁺, La³⁺, and mixtures thereof, and particularly desirable cations are Al³⁺, Ti⁴⁺, Zr⁴⁺, and mixtures thereof. The anion of the inorganic salt is not limited, as long as the inorganic salt has sufficient solubility in water. Examples of anions include, but are not limited to, chloride, bromide, nitrate, and sulfate.

The optional inorganic salt often can be present in a coating solution together with the optional surface crosslinking agent. The optional inorganic salt typically is present in a coating solution in an amount of about 0.5% to about 20% by weight, for example. The amount of optional inorganic salt present in a coating solution, and the amount applied to the base polymer particles, is related to the identity of the inorganic salt, its solubility in the solvent of the coating solution, the identity of the polyamine applied to the base polymer particles, and the amount of polyammonium carbonate applied to the base polymer particles. In general, the amount of optional inorganic salt applied to the base polymer particles is sufficient to form a tackless, monolithic polyamine coating.

In accordance with the present invention, the polyammonium carbonate and optional inorganic salt are applied to the base polymer particles in a manner such that each is uniformly distributed on the surfaces of the base polymer particles. Any known method for applying a liquid to a solid can be used, preferably by dispersing a coating solution into fine droplets, for example, by use of a pressurized nozzle or a rotating disc. Uniform coating of the base polymer particles can be achieved in a high intensity mechanical mixer or a fluidized mixer, which suspends the base polymer particles in a turbulent gas stream. Methods for the dispersion of a liquid onto the surfaces of base polymer particles are known in the art, such as that described in U.S. Patent No. 4,734,478.

Methods of coating the base polymer particles can include applying the polyammonium carbonate and inorganic salt simultaneously. The two components are suitably applied via two separate nozzles to avoid interacting before application to the surfaces of the base polymer particles. One suitable method of coating the base polymer is a sequential addition of the components. A more suitable method is a first application of the polyammonium carbonate, followed by an application of the optional inorganic salt. The resulting coated base polymer particles are then heated between about 70°C and about 175°C for sufficient time (e.g., about 5 to about 90 minutes) to generate the polyamine and to cure the polyamine coating.

As referenced above, the absorbent core 44 can also optionally include fluff, such as cellulosic fibers. Such fluff fibers may include, but are not limited to, chemical wood pulps such as sulfite and sulfate (sometimes called Kraft) pulps, as well as mechanical pulps such as ground wood, thermomechanical pulp and chemithermomechanical pulp. More particularly, the pulp fibers may include cotton, other typical wood pulps, cellulose acetate, debonded chemical wood pulp, and combinations thereof. Pulps derived from both deciduous and coniferous trees can be used. Additionally, the cellulosic fibers may include such hydrophilic materials as natural plant fibers, milkweed floss, cotton fibers, microcrystalline cellulose, microfibrillated cellulose, or any of these materials in combination with wood pulp fibers. Suitable cellulosic fluff fibers can include, for example, NB480, (available from Weyerhaeuser Co., a business having offices located in Federal Way, Washington U.S.A.); NB416, a bleached southern softwood Kraft pulp (available from Weyerhaeuser Co.); CR 54, a bleached southern softwood Kraft pulp (available from Bowater Inc., a business having offices located in Greenville, South Carolina U.S.A).; SULPHATATE HJ, a chemically modified hardwood pulp (available from Rayonier Inc., a business having offices located in Jesup, Georgia U.S.A.); NF 405, a chemically treated bleached southern softwood Kraft pulp (available from Weyerhaeuser Co.); and CR 1654, a mixed bleached southern softwood and hardwood Kraft pulp (available from Bowater Inc.)

The absorbent core 44 can be formed using methods known in the art. While not being limited to the specific method of manufacture, the absorbent core can utilize a meltblown process and can further be formed on a coform line. Exemplary meltblown processes are described in various patents and publications, including NRL Report 4364, "Manufacture of Super-Fine Organic Fibers" by V. A. Wendt, E. L. Boone and C. D. Fluharty; NRL Report 5265, "An Improved Device For the Formation of Super-Fine Thermoplastic Fibers" by K. D. Lawrence, R. T. Lukas and J. A. Young; and U.S. Patent Nos. 3,849,241 and 5,350,624.

To form "coform" materials, additional components are mixed with the meltblown fibers as the fibers are deposited onto a forming surface. For example, the superabsorbent materials of the present invention and fluff such as wood pulp fibers may be injected into the meltblown fiber stream so as to be entrapped and/or bonded to the meltblown fibers. Exemplary coform processes are described in U.S. Patent Nos. 4,100,324 to Anderson et al.; 4,587,154 to Hotchkiss et al.; 4,604,313 to McFarland et al.; 4,655,757 to McFarland et al.; 4,724,114 to McFarland et al.; 4,100,324 to Anderson et al.; and U.K. Patent GB 2,151,272 to Minto et al. Absorbent, elastomeric meltblown webs containing high amounts of superabsorbent are described in U.S. Patent No. 6,362,389 to D. J. McDowall, and absorbent, elastomeric meltblown webs containing high amounts of superabsorbent and low superabsorbent shake-out values are described in pending U.S. Patent Application Publication No. 20060004336 (Serial No. 10/883174) to X. Zhang et al.

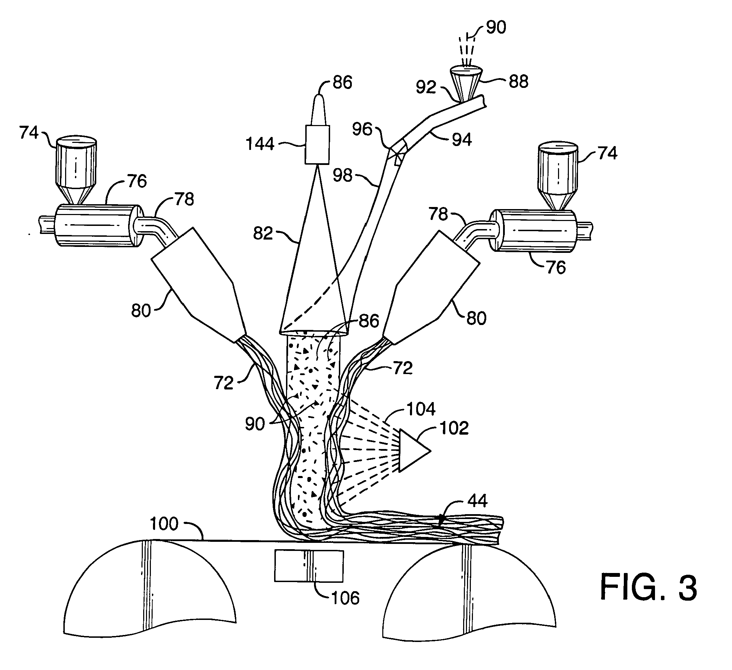

One example of a method of forming the absorbent core 44 of the present invention is illustrated in FIG 3. The dimensions of the apparatus in FIG 3 are described herein by way of example. Other types of apparatus having different dimensions and/or different structures may also be used to form the absorbent core 44. As shown in FIG 3, elastomeric material 72 in the form of pellets can be fed through two pellet hoppers 74 into two single screw extruders 76 that each feed a spin pump 78. The elastomeric material 72 may be a multicomponent elastomer blend available under the trade designation KRATON G2755 from Kraton Polymers, as well as others mentioned above. Each spin pump 78 feeds the elastomeric material 72 to a separate meltblown die 80. Each meltblown die 80 may have 30 holes per 2.5cm (inch, hpi). The die angle may be adjusted anywhere between 0 and 70 degrees from horizontal, and is suitably set at about 45 degrees. The forming height may be at a maximum of about 40.6cm (16 inches), but this restriction may differ with different equipment.

A chute 82 having a width of about 61 cm (24 inches) may be positioned between the meltblown dies 80. The depth, or thickness, of the chute 82 may be adjustable in a range from about 1.3 to about 3.18cm (0.5 to about 1.25 inches), or from about 1.9 to about 2.5cm (0.75 to about 1.0 inch). A picker 144 connects to the top of the chute 82. The picker 144 is used to fiberize the pulp fibers 86. The picker 144 may be limited to processing low strength or debonded (treated) pulps, in which case the picker 144 may limit the illustrated method to a very small range of pulp types. In contrast to conventional hammermills that use hammers to impact the pulp fibers repeatedly, the picker 144 uses small teeth to tear the pulp fibers 86 apart. Suitable pulp fibers 86 for use in the method illustrated in FIG 3 include those mentioned above, such as NB480.

At an end of the chute 82 opposite the picker 144 is a superabsorbent material feeder 88. The feeder 88 pours the superabsorbent material 90 of the present invention into a hole 92 in a pipe 94 which then feeds into a blower fan 96. Past the blower fan 96 is a length of 10.2cm (4-inch) diameter pipe 98 sufficient for developing a fully developed turbulent flow at about 1524m (5,000 feet) per minute, which allows the superabsorbent material 90 to become distributed. The pipe 98 widens from a 10.2cm (4-inch) diameter to the 61 cm (24-inch) by 1.9cm (0.75-inch) chute 82, at which point the superabsorbent material 90 mixes with the pulp fibers 86 and the mixture falls straight down and gets mixed on either side at an approximately 45-degree angle with the elastomeric material 72. The mixture of superabsorbent material 90, pulp fibers 86, and elastomeric material 72 falls onto a wire conveyor 100 moving from about 1.2m (14 feet) to about 10.7m (35 feet) per minute. However, before hitting the wire conveyor 100, a spray boom 102 optionally sprays an aqueous surfactant mixture 104 in a mist through the mixture, thereby rendering the resulting absorbent core 44 wettable. The surfactant mixture 104 may be a 1:3 mixture of GLUCOPON 220 UP and AHCOVEL Base N-62, available from Cognis Corp. and Uniqema, respectively. An under wire vacuum 106 is positioned beneath the conveyor 100 to assist in forming the absorbent core 44.

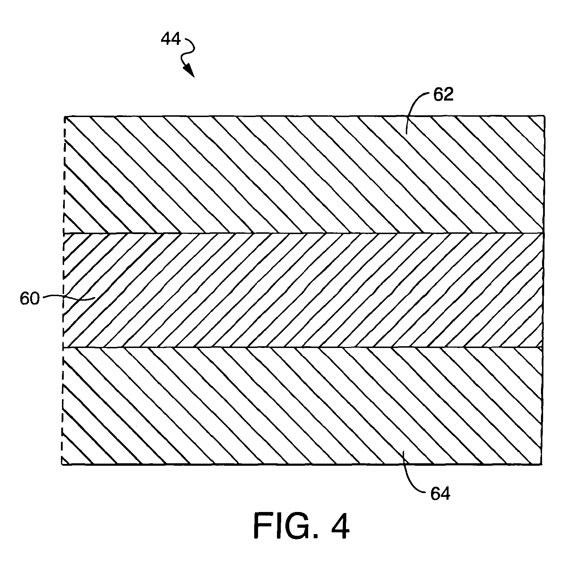

In general, the absorbent core 44 is often a unitary structure comprising a substantially uniform distribution of superabsorbent materials, fibers, and any other optional additives. However, referring to FIG 4, it has been discovered that the absorbent core 44 of the present invention may be further enhanced through structural modifications when combined with superabsorbent materials of the present invention. For example, providing a layer 60 comprising substantially superabsorbent materials of the present invention sandwiched between layers 62 and 64 comprising substantially fluff fibers, such as NB480, can result in an absorbent core 44 having improved absorbent properties, such as fluid insult intake rate, when compared to a structure comprising a substantially uniform distribution of the superabsorbent materials and fluff fibers. Such layering can occur in the z-direction of the absorbent core 44 and may optionally cover the entire x-y area. However, the layers 60 through 64 need not be discreet from one another. For example, in some aspects, the z-directional middle portion 60 of the absorbent core need only contain a higher superabsorbent material percentage (e.g., at least about 10% by weight higher) than the top layer 62 and/or bottom layer 64 of the absorbent core 44. Desirably, the layers 60 through 64 are present in the area of the absorbent core 44 that is within an insult target zone.

The present invention may be better understood with reference to the following examples.

### TEST PROCEDURES

### pH Test

One hundred milliliters (100 ml) of a 0.9% by weight sodium chloride (NaCl) solution are magnetically stirred at moderate speed in a 150 ml beaker without air being drawn into the solution. This solution is admixed with 0.5 ± 0.001 grams of the superabsorbent materials to be tested, and the resulting mixture is stirred for 10 minutes. After 10 minutes, the pH of the mixture is measured with a pH glass electrode, and the value is recorded only after it is stable, and, at the earliest, after 1 minute.

### Centrifuge Retention Capacity (CRC) Test

This test determines the free swelling capacity of a hydrogel-forming polymer. In this method, 0.2000 ± 0.0050 grams of dry superabsorbent material of size fraction 106 to 850 µm are inserted into a teabag. The teabag is placed in saline solution (i.e., 0.9 wt% aqueous sodium chloride) for 30 minutes (at least 0.83 I (liter) saline solution/1 g polymer). Then, the teabag is centrifuged for 3 minutes at 250 G. The absorbed quantity of saline solution is determined by measuring the weight of the teabag.

### Free-Swell Gel Bed Permeability (GBP) Test

This procedure is disclosed in U.S. Patent No. 6,387,495. The results are expressed in Darcies.

### 0.3 psi Gel Bed Permeability (GBP 0.3 psi) Test

This procedure is disclosed in pending U.S. Patent Application, publication No. 20050027268 (Serial No. 10/631916). The results are expressed in Darcies.

### Absorbency Under Load (AUL) Test

This procedure is described in EP-A 0615736. The results are expressed in g/g.

### Particle Size Distribution

A sample of superabsorbent material is added to the top of a series of stacked sieves, each of which has consecutively smaller openings. The sieves are mechanically shaken for a predetermined time, then the amount of superabsorbent material on each sieve is weighed. The percent of superabsorbent material on each sieve is calculated from the initial sample weight of the superabsorbent material sample.

### EXAMPLES

### Example 1

In this example, carbon dioxide was bubbled through a solution of CATIOFAST VHF (a polyvinylamine having a molecular weight of about 200,000 and a solids content of about 22% solids, available from BASF AG, Ludwigshafen, DE) to precipitate polyvinylammonium carbonate from the solution as fine particles. The resulting polyammonium carbonate dispersion was then used for application to surfaces of base polymer particles, as described below. Alternatively, the polyammonium carbonate can be filtered from the dispersion and applied to the base polymer particles as a solid, or can be diluted with a solvent (e.g., water or an alcohol) to faciltate application of the polyvinylammonium carbonate to surfaces of the base polymer particles.

40 grams of sodium polyacrylate base polymer having a Particle Size Distribution (PSD) of 106-850 µm (where the amount of particles greater than 850 µm is about 0.2 % by weight), a Centrifuge Retention Capacity (CRC) of 30 g/g and a Degree of Neutralization (DN) of 74 mol% were placed into a standard food processor at room temperature. The food processor was turned on and a first coating solution (Solution 1) comprising 0.66 grams of polyinylammonium carbonate (as active compound in a slurry, as described above), 2 grams of propylene glycol, and 2.4 grams of deionized water was injected into the food processor using a syringe and was allowed to coat the particles. Following the first coating solution, a second coating solution (Solution 2) comprising 0.8 grams of deionized water, 0.8 grams of propylene glycol, and 0.08 grams of ethylene glycol diglycidyl ether (EGDGE) was then injected into the food processor using a syringe and was allowed to coat the particles. The food processor was turned off, and the coated base polymer particles were removed. The coated particles were then placed in a BLUE M forced air laboratory oven (available from Thermal Product Solutions, a business having offices located in Montoursville, Pennsylvania, U.S.A.) at 150°C for 60 minutes to generate polyvinylamine by expelling carbon dioxide from the polyvinylammonium carbonate coating, and to cure the polyamine coating onto the base polymer particles, to form superabsorbent materials of the present invention. The superabsorbent materials were then tested for Centrifuge Retention Capacity (CRC), Absorbency Under Load (AUL), Gel Bed Permeability (GBP), and Particle Size Distribution (PSD) according to the test procedures described above, the results of which can be seen in Table 1 below.

**Table 1**

| **CRC (g/g)** | **AUL 0.9 psi (6.2kPa) (g/g)** | **Free Swell GBP (Darcies)** | **0.3 psi (2.1kPa) GBP (Darcies)** | **PSD >850 µm** |
|---|---|---|---|---|
| 23.2 | 18.6 | 235 | 13 | 0.9% |

### Comparative Example 1

In this example, 40 grams of sodium polyacrylate base polymer having a Particle Size Distribution of 106-850 µm (where the amount of particles greater than 850 µm is about 0.2 % by weight), a Centrifuge Retention Capacity of 30 g/g and a Degree of Neutralization of 74 mol% were placed into a standard food processor at room temperature. The food processor was turned on and a first coating solution (Solution 1) comprising 3 grams of CATIOFAST VHF (molecular weight of about 200,000 and a solids content of about 22% solids) and 2 grams of propylene glycol was injected into the food processor using a syringe and was allowed to coat the particles. Following the first coating solution, a second coating solution (Solution 2) comprising 0.8 grams of deionized water, 0.8 grams of propylene glycol, and 0.08 grams of EGDGE was injected into the food processor using a syringe and was also allowed to coat the particles. The food processor was turned off and the coated base polymer particles were removed. The coated particles were then placed in a BLUE M forced air laboratory oven at 150°C for 60 minutes to cure the polyamine coating onto the base polymer particles. The superabsorbent materials were then tested for Centrifuge Retention Capacity (CRC), Absorbency Under Load (AUL), Gel Bed Permeability (GBP), and Particle Size Distribution (PSD) according to the test procedures described above, the results of which can be seen in Table 2 below.

**Table 2**

| **CRC (g/g)** | **AUL 0.9 psi (6.2kPa) (g/g)** | **Free Swell GBP (Darcies)** | **0.3 psi (2.1 kPa) (Darcies)** | **>850 µm** |
|---|---|---|---|---|
| 24.7 | 16.6 | 206 | 6 | 16.7% |

Example 1 and Comparative Example 1 show that a polyamine coating comprising a polyamine derived from a polyammonium carbonate substantially reduces agglomeration compared to superabsorbent materials coated directly with a polyamine. In addition to reduced agglomeration (i.e., superabsorbent materials having a particle size greater than 850 µm), permeability is increased (both 0.3 psi (2.1 kPa) GBP and free swell GBP) and absorbency is substantially maintained (i.e., CRC and AUL 0.9 psi(6.2kPa)).

It will be appreciated that details of the foregoing examples, given for purposes of illustration, are not to be construed as limiting the scope of this invention. Although only a few exemplary embodiments of this invention have been described in detail above, those skilled in the art will readily appreciate that many modifications are possible in the examples without materially departing from the novel teachings and advantages of this invention. For example, features described in relation to one example may be incorporated into any other example of the invention.

Accordingly, all such modifications are intended to be included within the scope of this invention, which is defined in the following claims and all equivalents thereto. Further, it is recognized that many embodiments may be conceived that do not achieve all of the advantages of some embodiments, particularly of the preferred embodiments, yet the absence of a particular advantage shall not be construed to necessarily mean that such an embodiment is outside the scope of the present invention. As various changes could be made in the above constructions without departing from the scope of the invention, it is intended that all matter contained in the above description shall be interpreted as illustrative and not in a limiting sense.

## Claims

1. An absorbent article comprising:
a topsheet;
a backsheet; and
an absorbent core disposed between said topsheet and said backsheet; wherein
said absorbent core comprises a superabsorbent material that includes a base polymer; wherein
said base polymer has a surface coating that includes a polyammonium carbonate.

2. The absorbent article of claim 1 wherein said surface coating further comprises an inorganic salt having a polyvalent metal cation.

3. The absorbent article of claim 2 wherein said inorganic salt has a water solubility of at least 0.1 grams per 100 grams of water at 25°C.

4. The absorbent article of claim 2 or claim 3 wherein said polyvalent metal cation has a valence in the range of +2 to +4.

5. The absorbent article of any of claims 2 to 4 wherein said polyvalent metal cation is selected from Mg²⁺, Ca²⁺, Al³⁺, Sc³⁺, Ti⁴⁺, Mn²⁺, Fe²⁺, Fe³⁺, Co²⁺, Ni²⁺, Cu^{+/2+}, Zn²⁺, Y³⁺, Zr⁴⁺, La³⁺, Ce⁴⁺, Hf⁴⁺, Au³⁺, and mixtures thereof.

6. The absorbent article of any of claims 1 to 5 wherein said superabsorbent material is surface crosslinked.

7. The absorbent article of any of claims 1 to 6 wherein said base polymer comprises a plurality of pendant neutralized and unneutralized carboxylic acid groups.

8. The absorbent article of any of claims 1 to 7 wherein said base polymer is selected from acrylic acid, methacrylic acid, and combinations thereof.

9. The absorbent article of any of claims 1 to 8 wherein said base polymer has a degree of neutralization from about 25 mol% to about 100 mol%.

10. The absorbent article of any of claims 1 to 9 wherein said polyammonium carbonate is present on surfaces of said base polymer in an amount of about 0.1 % to about 2% by weight of said base polymer.

11. The absorbent article of any of claims 1 to 10 wherein said polyammonium carbonate is based on a polyamine comprising at least one selected from primary amino groups, secondary amino groups, tertiary amino groups, and quaternary ammonium groups.

12. The absorbent article of any of claims 1 to 11 wherein said polyammonium carbonate has a weight average molecular weight of about 5,000 to about 1,000,000.

13. The absorbent article of claim 11 wherein said polyamine carbonate is a homopolymer selected from polyvinylamine, polyethyleneimine, polyallylamine, polyalkyleneamine, polyazetidine, polyvinylguanidine, poly(DADMAC), cationic polyacrylamide, polyamine functionalized polyacrylate, and combinations thereof.

14. The absorbent article of claim 11 wherein said polyamine is a copolymer of polyvinylamine, polyethyleneimine, polyallylamine, polyalkyleneamine, polyazetidine, polyvinylguanidine, poly(DADMAC), cationic polyacrylamide, polyamine functionalized polyacrylate, or a combination thereof.

15. The absorbent article of any of claims 1 to 12 wherein said polyammonium carbonate comprises a homopolymer of polyvinylammonium carbonate.

16. The absorbent article of any of claims 1 to 12 wherein said polyammonium carbonate comprises a copolymer of polyvinylammonium carbonate.

17. The absorbent article of any of claims 1 to 16 wherein said base polymer comprises a polyacrylic acid.

18. The absorbent article of any of claims 1 to 17 wherein said base polymer is surface crosslinked.

19. The absorbent article of any of claims 1 to 18 wherein said superabsorbent material is heat treated.

20. The absorbent article of any of claims 1 to 19 wherein at least one of said topsheet, backsheet, and absorbent core is stretchable.

21. The absorbent article of any of claims 1 to 20 wherein said absorbent core comprises layers.

22. The absorbent article of claim 21 wherein at least one of said layers comprises substantially said superabsorbent material and at least one of said layers comprises substantially fluff.

23. The absorbent article of any of claims 1 to 22 wherein said article is selected from personal care absorbent articles, health/medical absorbent articles, and household/industrial absorbent articles.

24. The absorbent article of any of claims 1 to 23 wherein said absorbent core comprises at least about 10% by weight of said superabsorbent materials.

25. The absorbent article of any of claims 1 to 23 wherein said absorbent core comprises at least about 30% by weight of said superabsorbent materials.

26. The absorbent article of any of claims 1 to 23 wherein said absorbent core comprises at least about 60% by weight of said superabsorbent materials.

27. The absorbent article of any of claims 1 to 23 wherein said absorbent core comprises between about 10% and about 80% by weight of said superabsorbent materials.

28. The absorbent article of any of claims 1 to 27 wherein said absorbent core further comprises fluff.

29. The absorbent article of any of claims 1 to 28 wherein said absorbent core is treated with a surfactant.

30. A method for surface coating a superabsorbent material with a polyamine, comprising surface coating the superabsorbent material with a polyammonium carbonate and then converting the polyammonium carbonate into a polyamine.

31. A method according to claim 30, wherein the polyammonium carbonate is converted into a polyamine by heating.

32. A method according to claim 30 or claim 31, wherein the polyammonium carbonate is formed by treating a polyamine with carbon dioxide.
